# EUROPEAN PATENT APPLICATION

(11) **EP 1 741 467 A1**
(43) Date of publication of application: **10.01.2007**
(21) Application number: 05721991.7
(22) Date of filing: 01.04.2005
(51) Int. Cl.: A61N 2/02

(54) **ELECTROMAGNETIC APPARATUS FOR THE TREATMENT OF LESIONS ASSOCIATED WITH INADEQUATE BLOOD PERFUSION, PARTIAL DENERVATION, TISSUE LOSS, PAIN, OEDEMA, INFLAMMATION AND INFECTION**

(30) Priority: 01.04.2004 MX PA04003115
(71) Applicant: Canedo Dorantes, Luis, C.P. 62490 Cuernavaca, Morelos (MX)
(72) Inventor: Canedo Dorantes, Luis, C.P. 62490 Cuernavaca, Morelos (MX)
(74) Representative: Viering, Jentschura & Partner
(86) International application number: PCT/MX2005/000020
(87) International publication number: WO 2005/094940

(57) **Abstract**

The invention relates to an apparatus for treating a bodily lesion that is associated with inadequate blood perfusion, partial denervation, neuronal apoptosis, tissue loss, pain, oedema and/or infection. The inventive apparatus is used to apply external, non-invasive, analgesic electromagnetic fields (EMF) to the patient with the lesion, in an area removed from the site of the lesion. The aforementioned electromagnetic fields are effective in increasing angiogenesis, vasculogenesis, neural tissue regeneration and osteogenesis, restoring the wound repair process, providing analgesic, anti-oedema, anti-inflammatory effects and/or repairing wounds. The electromagnetic fields include frequencies from several Hertz up to less than approximately 300 Hz, as well as components of a static field from between several microteslas and a maximum intensity of between approximately 0.3 and approximately 0.8 mT, which can be applied alone or together with a homogeneous static field from approximately 40 to approximately 80 T, or approximately 400 to approximately 800 Gauss.

## Description

### FIELD OF THE INVENTION

This invention refers, in general, to a method and electromagnetic device for the treatment of cutaneous injuries and internal injuries, for example, hypoperfused but still viable myocardium (hibernating myocardium). More specifically, this invention is associated with a method and device for the treatment of patients suffering from inadequate blood perfusion, loss of tissue, partial denervation, injuries, bone fractures, burns and/or ulcers; consisting in the single application of time-variable magnetic fields or the simultaneous application of time-variable magnetic fields and static fields on a subject with specific characteristics.

### BACKGROUND OF THE INVENTION

It has been observed that the local application of electromagnetic fields (EMF) on rats promotes the regeneration of the skin and the nerve tissue, intensifies the maturation of the collagen and increases the tensile strength during the healing process of cutaneous injuries. In vitro tests have proved that electromagnetic fields also induce the proliferation and production of collagen-mediated fibroblast and indirect angiogenesis. In addition, it has been known for some time now that the local application of EMFs promotes the healing of soft tissue and nerve regeneration, reduces edema, inflammation and pain. Ulcers in legs caused by chronic venous failure and ulcers caused by pressure (eschars) have been treated by the application of several therapies including the local application of growth factors, hyperbaric oxygen therapy (HBO), infrared light irradiation, EMF, ultraviolet light irradiation and low power laser as well as ultrasound waves, directly on the ulcerated region. Nevertheless, the Agency for Health Care Policy and Research of the U.S. Department of Health and Human Services has recommended the local application of EMFs as the only supplementary therapy with enough support evidence for the treatment of ulcers caused by pressure. Although the local application of EMFs has been used to treat leg ulcers caused by chronic venous insufficiency, to regenerate nerve tissue, to treat complications arising from the non-union of bones and to protect animal models from the sequels of induced ischemia; all current attempts to achieve a cure through the application of EMFs on a point distant from the affected region have been unsuccessful.

The ability to treat wounds and other ailments by applying EMFs on a zone away from the region of the wound and achieve an improvement or even the total healing of the wound would be extremely useful, particularly in cases where the wounded region is not accessible or disturbing the patient is not desirable. This type of treatment could be given to ambulatory patients with the device object of this invention, it would not require highly qualified personnel and would reduce the cost of the treatment and at the same time it would free medical-qualified personnel to focus on the diagnostic and prescription of therapies and to follow up the results obtained in any series of particular applications.

Therefore, there is an obvious need for an improved method and device that speeds up and promotes the healing of wounds on people, particularly those wounds that have proved to resist other more conventional treatments; a method and device that is easy to use, that does not disturb the patient, that can be used on ambulatory patients by personnel who does not need to be highly qualified and is comfortable for the patient.

There is a need for a highly beneficial method and device for electromagnetic treatment.

### BRIEF DESCRIPTION OF THE INVENTION

This invention refers to a method to treat injuries of several tissues, burns, cutaneous ulcers and hypoperfused but still viable myocardium, including the external, non-invasive application of low frequency electromagnetic fields (EMF) with specific characteristics, in a zone distant from the site of the injury, on a person suffering from a burn or ulcer, applied effectively with the purpose of accelerating the neuronal apoptosis, nerve regeneration, the activation of stem cells, heal wounds due to burns, heal bone injuries or injuries caused by ulcers, amplify the angiogenesis and vasculogenesis of different tissues and to reinforce the effect of other simultaneously applied therapies. The adequate EMFs include an electromagnetic field component that is time variable and is generated at frequencies ranging from a few Hertz (more than one) to less than approximately 300 Hz; and static field components with a strength of nearly a few micro Teslas (µT), for example, in the interval of approximately 2 - 5 µT, approximately 20 µT to approximately 100 µT, also approximately 0.3 mT to approximately 0.8 mT. These EMFs can be applied individually or in combination with a homogeneous static field of approximately 40, approximately 50 to approximately 70, approximately 80 µT (or approximately 400, approximately 500 to approximately 700, approximately 800 Gauss). Although in some cases the measured frequencies inside the coils range from approximately 0.3 to approximately 0.8 mT, in those areas of the skin located close but not inside the coils, the strength of the EMFs is reduced progressively to environmental magnetic fields level.

The method and device of this invention is adapted, particularly, for the therapeutic treatment of persons suffering from cutaneous wounds, for example, burns, internal wounds such as bone fractures, partial denervation and ulcers, specially chronic wounds, for example, leg ulcers due to venous and arterial failure, ulcers due to pressure, open wounds, infected wounds, swollen painful tissues, areas with inadequate blood perfusion such as hypoperfused but still viable myocardium, decaying blood vessels, cardiac muscle and striated muscle, decay of the cells of the central and peripheral nervous system and similar afflictions, which are present by themselves or associated to other causes, for example, atherosclerosis, varicose veins, diabetes, hypertension, rheumatoid arthritis, trauma and the like. Some wounds resist or are impervious to treatment with surgical methods or conservative, conventional methods; nevertheless, the application of the therapy of this invention makes them highly sensitive to the treatments that were previously ineffective. This method can be applied during both, a short and an extended period of time, by periodically subjecting a patient that has already received the treatment to repeated treatments during an effective period of time to reduce the intensity of pain, edema, inflammation and infection, and to increase angiogenesis, vasculogenesis, regeneration of the nerve tissue, muscle tissue, bone union and to stimulate the immune system and the healing process of the wounds in different tissues.

One of the purposes of this invention is to provide a method to deliver electromagnetic fields that will greatly reduce the healing time in persons who had shown great resistance to preliminary conventional medical treatments.

Another purpose of this invention is to produce a device that emits extremely low frequency magnetic fields.

Another purpose of the invention is to provide a device that emits extremely low frequency magnetic fields to be delivered to a patient to increase his or her collateral circulation.

Another purpose of this invention is to provide a device that emits extremely low frequency magnetic fields, suitable for a specific area of the patient's body, namely a part such as an arm, a hand, the thorax, a foot, a leg.

Still another purpose of this invention is to provide a device that emits extremely low frequency magnetic fields with specific components, both constant and variable, that can be applied advantageously on an area distant from the wound.

It is also a purpose of this invention to provide a device that emits extremely low frequency magnetic fields that stimulate the immune system, preferably the peripheral blood mononuclear cells (PBMC).

### BRIEF DESCRIPTION OF THE DRAWINGS

There are two preferred ways to expose a part of the body to the magnetic fields of this invention. The first form uses a design that combines time-variable magnetic fields with static fields. Several forms of the device of this invention are provided, portable or fixed, for application on the arm or thorax.

Figure 1 is a top view of the parallel pair of Helmholtz coils, along with the independent elongated rail-type base that serves as a fastening element and for horizontal motion.

Figure 2 is a perspective view of the device of figure 1, where the pair of coils is shown along with its base and the independent rail for motion.

Figure 3 is a lateral view of figure 2, where the horizontal motion wheelbarrow for the coils is shown.

Figure 4 is a cross section view of figure 2, where the section of the base of the coils and the motion rail is shown.

Figure 5 is a perspective view of the complete device, where the rectangular plate and the outer shell that fastens it and covers the wheelbarrow for the coils is shown.

Figure 6 is a cross section view of the complete device of figure 5 that shows the section of the coils, the rectangular top plate and the outer shell.

Figure 7 is a top view similar to figure 1, but with an additional rectangular plate where the patient's body lies.

Figure 8 is a lateral view of the device of figure 5 that shows the handle that connects both coils, the longitudinal lateral slot of the shell that allows the coils to move.

Figure 9 is a perspective view of a portable embodiment of this invention, adapted for a body part such as a leg or arm.

Figure 10 is a top view of the device of figure 9, that shows the two round Helmholtz coils, the four motion wheelbarrows, the fastening and push handle along with its control console and the base for placing the body part.

Figure 11 is a cross section view of the device of figure 6 that shows the wide and thin fastening stand that slides upwards and downwards that lays on a flat round base with four carts.

Figure 12 is a lateral view of another portable form of the device of this invention, where a part of the body such as arm, hand, leg or calf is placed.

Figure 13 is a perspective view of the device of figure 12 with a stand that has three bars that slide upwards and downwards to place the limb in a comfortable position, said bars have a flat and round base, at the lower part.

Figure 14 is a top view of another embodiment of the EMF device having a pair of round Helmholtz coils and a flat plate with rounded ends.

Figure 15 is a perspective view of the device of figure 14 with two stands at the ends, with bases on the elliptical section.

Figure 16 is a cross section view of the device of figure 15 that shows the round section of the coils, the flat section of the plate and the stand and the section of the base of the stands.

Figure 17 is a lateral view of the device of figure 15 that shows both parallel coils, the location handle between both, the section of the plate and the fastening stands that rest on the elliptical bases.

Figure 18 is a perspective view of the round coils with its fastening and sliding, longitudinal structural system.

Figure 19 is a cross section view of the coils with its fastening and sliding structure.

Figure 20 is a top view of the coils, the upside down handles to place the limb in a comfortable position; the bars have a flat and round base in the lower part.

Figure 21 is a lateral view of the coils, the fastening handles and the sliding crossbars.

Figure 22 is a perspective view of the base of the plate, the flanges that pair with the coil structure, the stands and bases on which the whole system of emission of electromagnetic fields of this invention is located.

Figure 23 is a lateral view of the base of the plate, the coupling with the stand and the base that rests on the floor.

Figure 24 is a top view of the base of the plate that shows the coupling flange with the sliding structure of the coils in detail.

Figure 25 is a lateral view of the base of the plate, the stands on which it is located and its respective bases that rest on the floor.

The objectives and special features of this invention will be observed more accurately through the following detailed description of the invention and the enclosed drawings.

### DETAILED DESCRIPTION OF THE PREFERRED FORMS

The method and device of this invention developed due to the interest of the inventor in improving the methods of the prior art and several therapeutic treatments that have been insufficient up to this date, said treatments also require the direct application on the injured zone, which makes them complex, difficult to carry out and definitively inconvenient for the patients.

The inventor surprisingly discovered that, when a patient with chronic cutaneous injuries and/or internal wounds, for example bone fractures, partial denervation or still viable hypoperfused myocardium, undergoes a treatment by applying an electromagnetic field (EMF) to a part of the body, according to this invention, the wounds show an activation of the inflammatory reaction and healing, drastically reducing the extent of the wounds with minimum effort, in many cases, the wounds healed completely and even scarred easily or the wound was completely eliminated. This occurs even in the event of wounds that resist other generally effective therapies. In patients with hypoperfused but still viable myocardium, the application of EMFs to a part of the body according to this invention, produces an increase of the collateral circulation and myocardium contraction, resulting in an adequate clinical recovery. The beneficial results obtained by the inventor are achieved by applying specific electromagnetic fields (EMF) with specific components both constant and variable, to a part of the body of the patient, such as arm, thorax, hand, leg, foot or even the whole body. Clinical experience shows that some patients with internal and/or external wounds become resistant to drugs or other generally effective therapies administered to heal wounds after a certain period of time. Besides, there are other injuries, internal wounds, hypoperfused tissues, burns and ulcers that do not respond to these treatments.

The inventor hereof has surprisingly found that, when those patients are exposed to external, non-invasive electromagnetic fields (EMF) produced by the devices of this invention, they respond to the previous treatments and drugs to treat wounds that were ineffective before. Many patients who could benefit from this treatment have a history of serious cardiac arrest, leg ulcers caused by venous or arterial failure, burns, ulcers caused by pressure, infected surgical wounds, bone fractures, loss of tissue, partial denervation, inadequate blood perfusion and swollen and painful soft tissue, among other illnesses, that are resistant or impervious to available surgical or pharmacological methods or other conventional treatments.

This invention uses specially configured, low frequency electromagnetic fields (EMF) produced by the device of this invention that can be advantageously used on a zone distant from the wound. The EMFs of this invention stimulate the immune system of the patient, preferably, the activated and memory peripheral blood mononuclear cells (PBMC) of the patient, although the action of the EMFs on other exposed bodily tissues is not excluded. The activation of the immune system of the patient causes a natural response that provides the cells, cytokines, angiogenic, osteogenic and neural factors, growth factors, hormones and other necessary immunomodulators that motivate the therapeutic healing of wounds, such as burns, leg ulcers caused by venous and arterial insufficiency, ulcers caused by pressure, chronic infected wounds, internal or external areas with inadequate blood perfusion, for example hypoperfused but still viable myocardium and the like; loss of muscle, bone fractures, partial denervation, swollen, painful soft tissue and other cutaneous injuries; to the zone of the injury and other areas of neuroendocrine regulation of the immune response. The electromagnetic fields (EMF) of this invention include time-variable electromagnetic fields, for example, alternate currents that oscillate at prescribed frequencies. In an embodiment of this invention, the alternate currents are generated in a solenoid with frequencies that oscillate from a few Hertz (more than approximately 1 cycle per second) to approximately 10 Hertz, from approximately 50 Hertz to approximately 100 Hertz; from approximately 200 Hertz to approximately 300 Hertz, with maximum intensities of about 120 Hertz and a harmonic resonance. These time-variable magnetic fields have small associated static fields, that range from some micro Teslas (µT), for example, approximately 2-5 µT, from approximately 10 µT to approximately 100 µT, from approximately 0.3 mT to approximately 0.8 mT. The static fields with an intensity of approximately 40, approximately 50 to approximately 60 µT, approximately 70 µT, approximately 80 µT (400 to 800 Gauss), are generated by the motion of electrons around atoms and electrons in crystalline meshes inside permanent magnets used in the devices of this invention located around the solenoid or in the Helmholtz coils.

The device of this invention achieves the healing of the damaged tissue either directly or in combination with immunocytes or other exposed tissues, or through substances produced by the cells, reducing the pain, edema and infection while restoring or stimulating the signaling network regulated by the immune response of the patient, which is necessary to increase the angiogenesis, vasculogenesis and healing of the wound. When a part of the body, for example an arm, or the whole body is exposed to the EMFs produced by this invention, the PBMCs as well as other tissues, for example, skin, muscle, lymphatic and blood vessels, erythrocytes, bone, nerve tissue and extra cellular matrix, are under the effect of the EMFs produced by the devices of this invention. Under this conditions, the exposed tissues are simultaneously stimulated to secrete products that act off-site: on the wound, hypoperfused tissue, partially denervated area, healing of bone fractures or on the neuroendocrine centers that regulate the immune response, contributing to regulation of the healing of the wound.

This way, the present invention is associated with a method and a device to treat a body injury associated with pain, edema, inflammation, bone fractures, partial denervation and/or infection, damaged wound repair mechanism and/or hypoperfused tissue, for example hypoperfused but still viable myocardium and the like, inadequate blood perfusion in the area of the wound and/or that show a inflammatory response regulated by the immune system and a process of healing wound damaged tissues; which includes the external, non-invasive and remote application of effective electromagnetic fields (EMF) to a patient suffering from a wound.
These electromagnetic fields have an analgesic, angiogenic and vasculogenic effect; they promote the growth of nerve tissue, reduce edemas and heal the wounds effectively.
These EMFs include frequencies that range from a few Hertz, preferably from approximately 0.1 Hz, approximately 1 Hz, approximately 5 Hz, approximately 10 Hz, approximately 20 Hz, approximately 60 Hz, approximately 80 Hz, approximately 100 Hz, approximately 120 Hz to less than approximately 180 Hz, approximately 240 Hz, approximately 300 Hz; and components of a static field with a minimum intensity that ranges from a few micro teslas, for example, from approximately 2-4 µT, approximately 10 µT, approximately 50 µT, approximately 100 µT, to approximately 0.3, approximately 0.4, approximately 0.5, approximately 0.6, approximately 0.7, approximately 0.8 mT. The EMFs can be applied individually or combined with a static field of approximately 40, approximately 50 to approximately 70, approximately 80 µT (or approximately 400, approximately 450, approximately 500, approximately 550 Gauss to approximately 600, approximately 650, approximately 700, approximately 800 Gauss).

In this invention, the device that produces the EMFs has been adapted to apply the fields to the limb of a patient: an arm or a leg, and in some cases a hand or a foot, the thorax or other bodily parts of the patient, even to the whole body. The electromagnetic field can be applied for several periods of time, ranging from approximately 5 minutes to approximately 7 hours per application. In a preferred form, the use of electromagnetic fields (EMF) is carried out over an effective period of time to expose the entire blood volume of the patient to the EMFs at least once. This procedure guarantees that the entire blood volume is exposed to the EMFs and the inductive effect reaches all the cells present in the blood stream, particularly the activated and memory peripheral mononuclear blood cells and their mother cells.
Although in most cases a single application of the electromagnetic fields (EMF) is enough, the application can be repeated as many times as necessary. Preferably once a week, two times a week, 3, 4, 5, 6 and 7 times a week and up to approximately 1,2, 3, 4, 5, 6, 7 and 8 weeks and even a few months and years. The method also includes the periodic re-application of the EMFs as well.

The electromagnetic fields of the present invention can be applied as adjuncts for the treatment of illnesses and conditions associated or caused by bodily injuries or to those causing bodily injuries. In all cases, a sufficient number of applications of electromagnetic fields (EMF) can be delivered until the wound heals completely. The injuries that can be treated with the device of this invention include chronic injuries, partial denervation, loss of muscle tissue, bone fractures, burns, injuries caused by inadequate blood perfusion, for example, hypoperfused but still viable myocardium and the like; the injuries arising from an inadequate surgery and of different types of venous, arterial and vascular problems, including arterial and venous ulcers.

The application of electromagnetic fields is adequate to increase the effect of a treatment applied to a bodily injury associated with pain, edema and/or inflammation. In many cases, a patient becomes more receptive to a treatment that was previously ineffective after a series of electromagnetic field applications. This way, the device and the method of this invention increase the response to the treatment, which, at the same time, results in the healing of the bodily injury and the relief of the associated pain, edema and/or inflammation.

The method and device of this invention is particularly valuable for patients who have become resistant to any agent used to treat a disorder or condition because the application of the electromagnetic fields (EMF) restores the ability of the pharmaceutical or surgical treatments to improve the health and mobility of the patient. Some examples of this type of treatments are: angioplasty, surgery, saphenectomy, skin grafting, closing of the wound, stitches, bandages, treatment with antihypertensive drugs, use of pharmaceutical compositions, nurse care and total cleaning of the wound, among others. Some examples of pharmaceutical compositions that are typically delivered to patients suffering from the aforementioned types of injuries include pain blockers, anti-inflammatory agents, antibiotics, flavonoids, tissue plasminogen activator, acetylsalicylic acid, ketanserin, zinc oxide, hydrocolloids, alginates, foams, hydrofibers, gels, collagen mixtures, silver, compression systems, prostacyclin analogues, prostanoids, venotonic drugs, fibrinolytic and thrombolytic agents, dieting and the like. These agents can also be delivered at the same time or after the delivery of EMFs, according to this invention.

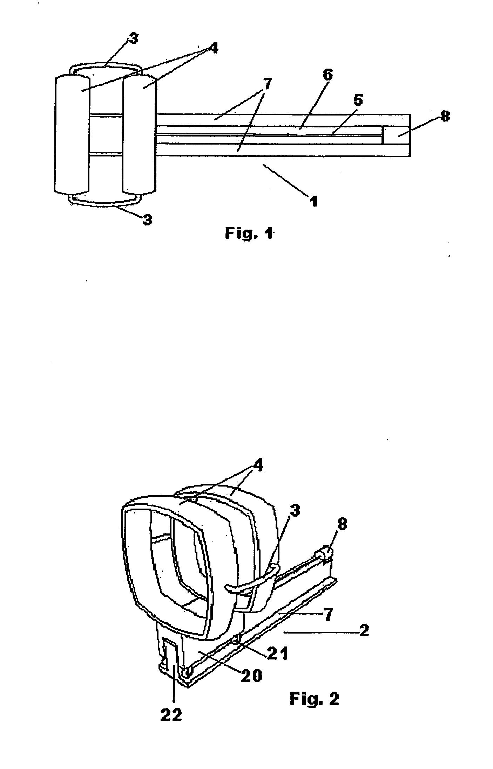

The preferred forms of the invention, mentioning the drawings as to facilitate the understanding of this invention, are described below. This treatment can be delivered with the device of the invention illustrated as an example in figures 1 through 17. The designs of the several embodiments of this device derive from the observations of patients with similar problems who were exposed to the time-variable magnetic fields of this invention either individually or combined with static fields.

In the preferred embodiment, which has been used many times with success, the permanent magnets are eliminated but the time-variable electromagnetic fields are kept. Another form provides a device with enveloping coils that form an internal chamber big enough to expose a part of the human body to an electromagnetic field inside the chamber, for example from the upper limit of the aorta to the lower limit of the liver and spleen. This way, time-variable magnetic fields with the same characteristics can be delivered to the major blood and lymphatic vessels: aorta, vena cava and thoracic duct, as well as the major blood and lymphatic deposits: heart, lung, liver, spleen and paravertebral lymph node of the patients. This treatment simultaneously exposes a great volume of peripheral mononuclear blood cells to the electromagnetic fields and reduces the duration of the treatment. These forms of the device were designed to provide the patient with a comfortable position during the exposure to the magnetic fields.

In yet another form of the device of the invention, the characteristics of the time-variable magnetic fields are the same than in the one described above, but are produced by, for example, two parallel coils, for example, Helmholtz coils. The coils produce electromagnetic fields, the field lines of which are located on the internal space of the coils. This mode can be used to expose a bodily part or the whole body to the lines of the magnetic field variable in time that run parallel to the axis of the body, with the patient lying down on a horizontal plate, made of non-magnetic material.

In other additional forms of the invention, the electromagnetic fields of this invention can be generated in two different ways. One way consists of generating the electromagnetic fields by modulating continuous waves of approximately 27.12 MHz that carry electric and magnetic energy through space (transmitting wave) at the same frequencies or similar frequencies to those of this invention (for example, an antenna to concentrate the electromagnetic fields of this invention in any direction). The other way is by modulating high intensity and frequency electromagnetic fields of (transmitting waves) similar to the electromagnetic fields produced by a Tesla coil, at frequencies equal or similar to those employed in the present invention. In this last example, the electromagnetic fields traveling through space can reach a patient lying down on a bed or seated, who does not need to be connected to the device.

The embodiments of this invention allow a bodily part or the whole body to be exposed to the EMFs, as shown in figures 1 through 17. In this design, the main blood vessels (aorta and vena cava), the thoracic duct, the paravertebral lymph nodes and the main blood deposits (liver, spleen, lung and heart) could be simultaneously exposed to the EMFs (not shown), reducing the patient exposure time.

Making reference to figure 1, which shows the top view of the Helmholtz coils alignment that constitutes the device (1) of this invention, the device consists of a pair or parallel coils (4) with 625 turns each and with a shell made of non-magnetic material shaped like a curve-sided quadrilateral having a diameter of 80 cm, the coils are separated 40 cm from each other, said coils have a common handle (3) at one of their vertical sides thereby joining them together and works as a fastening handle so that the operator may slide the coils horizontally to the adequate position so as to irradiate the limb or part of the body to be exposed to the magnetic field. The coils are mounted over a sliding base, not shown, which moves all along the horizontal surfaces (7) or flanges of the rail; following the base there is a central longitudinal guiding slot (5) located at the center of the top flat longitudinal surface (6); at the opposite end of this surface (6) there is a distal stop (8) that prevents the coils (4) from sliding beyond the space delimited by the surface (6); the surfaces (6) and the flanges (7) are part of a longitudinal rail-type sliding base on which the cart that supports the Helmholtz coils producing low frequency magnetic field lines, moves. The device of this invention generates very low frequency electromagnetic fields both variable in time and static, ranging from 1 up to 300 Hz, with intensities ranging from 2 micro teslas up to 0.8 militeslas; said fields are delivered during several periods of time which should be enough to expose the entire blood volume of the patient. Said electromagnetic fields have very low frequencies and they are delivered locally to rehabilitate and promote the regeneration of skin and nerve tissue.

Figure 2 shows a perspective view (2) corresponding to the elements of figure 1, where the Helmholtz coils (4) with their respective positioning handle (3) are located on the rectangular sliding base (20) and the central inferior inverted horseshoe-shaped opening, which, in turn, is mounted on four wheels (21) located on the lower edges of each one of the lateral sides of the base, thereby forming a sliding cart that moves horizontally when the operator pulls it using the handle (3); this motion takes place on the flat horizontal surface (7) of the flanges of the longitudinal rail-type base (22); the rail, the coil base and the wheels are manufactured of a non-magnetic material. The operator can slide the coils horizontally to the left and to the right, so that he or she may place the coils in the adequate position to treat the illness.

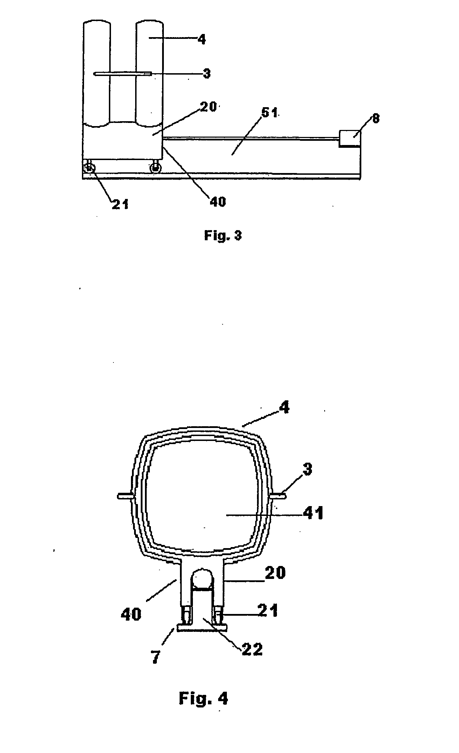

Figure 3 is a lateral view of the device, not showing the plate. Thickness (50) of the base of the rail and height (51) are defined, as well as the section of the distal stop at the end (8), the set of coils (4), handle (3), the base (20) of the coils and the wheelbarrows (21) that form the cart (40) that moves longitudinally across the base.

Figure 4 is a cross section view of the position of the coils in figure 2, that shows the hollow space or tunnel (41) delimited by the coils; the plate (30) of figure 3 is located in that space; it also shows the position of the sliding cart (40) formed by the base (20) of the coils and wheelbarrows (21) joined to the lower section of the base and sliding across the surfaces (7) of the rail (22) thanks to the pulling action on the handles is clearly delimited.

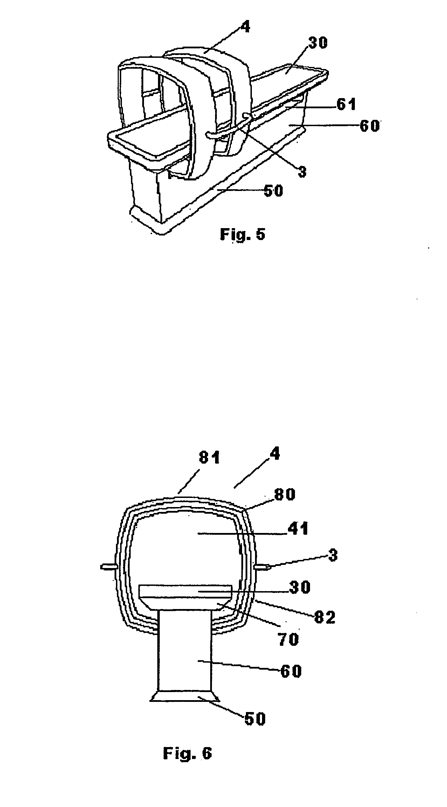

Figure 5 is a perspective view of the device of this invention that shows the cart (40) and the rolling surface (7) covered by a rectangular prism-shaped shell (60) with a top longitudinal slot (61) on the elongated lateral sides; this slot delimits a lateral space where the set of Helmholtz coils slides (4) to cover, upon moving, part of the space of the top plate (30) where the patient lies down; the outer shell (60) lays on the thickness of the base of the rail (50). The plate (30) is longer than the base of the rail and exceeds the limits defined by the outer shell, so that the patient lies down comfortably. The device can be manufactured of wood, plastic or other non-magnetic material, to avoid any interference with the electromagnetic field to be applied to a body part of the patient.

Figure 6 is a cross section view of the fully assembled electromagnetic field device; the coils (4) are formed with four curved sides, two vertical (82) and two horizontal (81); the sides are joined at four rounded edges (80), thus forming the empty space (41) where the magnetic field lines generated by the coils pass through; the body or limb of the patient to be exposed to the flow of magnetic field lines is also placed in this space; the patient lies down on the plate (30) and the coils manually slide in a direction parallel to the body of the patient The plate (30) and its base (70) with sloping edges are within the internal space delimited by the coils; the plate lays on the outer shell (60) and the whole device lies on the floor with the help of the base (50).

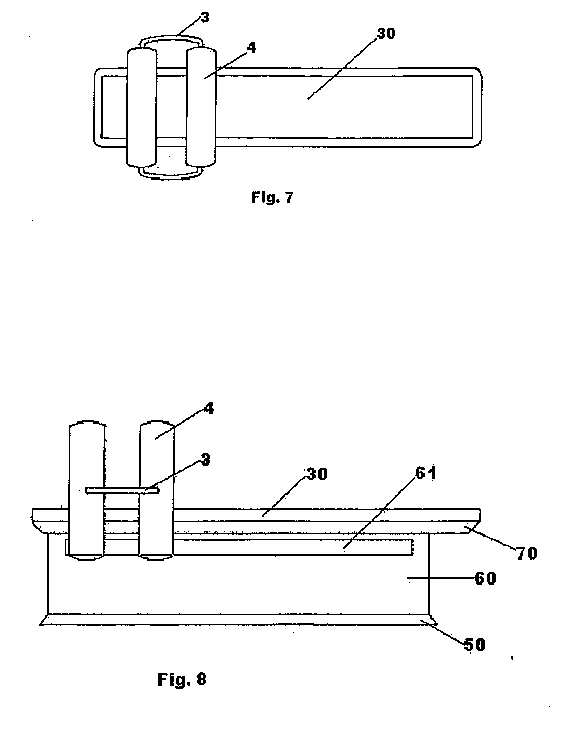

The device described in figures 1 and 2 also has a plate (30) made of non-magnetic material that is shown in the top view of figure 7; this plate is located above the base of the coils (4) and passes through the internal space delimited by the coils. The body of the patient lies down horizontally in a comfortable position on the plate, using the handle; the coils are placed at the proper position (3) to irradiate the patient with the magnetic field lines generated by the coils.

Figure 8 is a lateral view of the device of figure 5 that shows the location of the outer shell (60) in relation to the base of the rail (50) and the location of the longitudinal lateral slot fitted out to allow the passage of the lower side of the coils and to carry out the horizontal sliding movement of the coils; the thickness of the plate (30) and the base (70) with sloping edges that supports the plate is also shown. The coils can travel all along the horizontal distance permitted by the slot (61). The plate and its respective base are longer than the length of the shell; the plate extends beyond the limits delimited by the edges of the shell that covers the rail-type base.

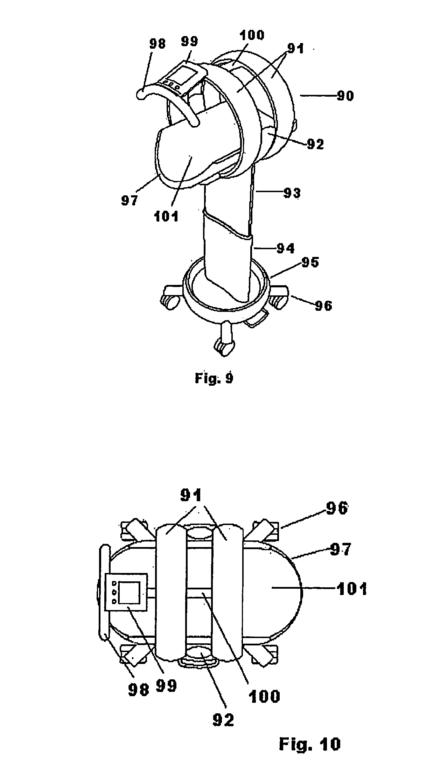

Figure 9 is a perspective view of another embodiment of this invention where the device is a portable version (90), the patient can place it on a limb, such as an arm or leg, without moving from where he or she is; this way, patients that for some reason are unable to move can be exposed to the magnetic fields. This embodiment consists of two circular, doughnut-shaped Helmholtz coils (91); the coils are held by a semicircular ring (92) that is located parallel to the coils and between their inner edges (the space); the ring is held by a flat and wide telescopic stand divided in two sections, the upper section (93) that can slide inside the thicker, lower section (94) joined to the round and flat circular base (95) with ring-shaped edges, that, at the same time, is mounted on four wheelbarrows (96) distributed at uniform angular distances on the outer surface of the ring that constitutes the base. In the interior of the coils there is a space that houses a flat and concave shaped support (97), with two straight edges and two ends shaped like the section of an ellipse on which the limb of the patient to be irradiated by the magnetic field is placed; another fixing point is located in the upper part and the internal surface of the coils; the fixing point is made out of a long and perpendicular fixing element attached to the coils; the control panel (99) and the displacement handle are located at the outer end of this fixing element; this support (100) is long and perpendicular to the coils, it extends beyond one of the outer edges of one of them; in that extension there is a square-shaped control panel (99) with a screen; at the end there is a round semicircular handle (98) that is used to move the device by applying force on it. The device is located at the adequate height and distance so that the patient laying down or seated is irradiated by the magnetic field generated by the coils; the patient places the limb (arm, foot, hand or leg) on the surface (101) and the device is turned on.

Figure 10 is a top view of the device of figure 9 that shows a close-up of the two round coils (91) in a Helmholtz (parallel) configuration; the support (100) placed on the internal surface of the coils holds the control panel (99) and the displacement handle (98). The support surface for the limb (97) when viewed from the top, has a concave surface (101) with a semi-elliptical shape, the intermediate support (92) between the coils holds the device to the base, which in turn, is connected to the four wheelbarrows (96) that move the group to the target by pushing the handle (98) located at the end of the top part of the device.

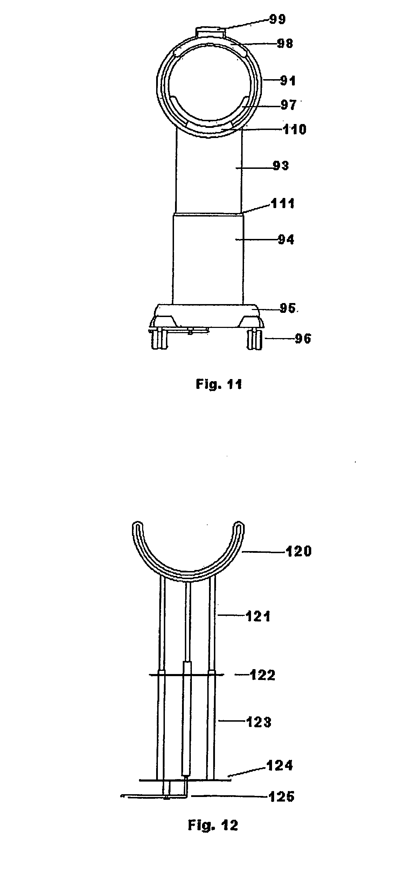

Figure 11 is a lateral view of the device of figure 9 that shows the control panel (99), the displacement handle (98), the Helmholtz coils (91), the support (97), the tray (110) support (97); this group is supported by the flat stand (93), which can slide through the opening (111) to reach a comfortable height for its use; the flat stand (94) rests on the round base (95) and the whole device is mounted on four wheelbarrows (96) that move said device.

Figure 12 is a lateral view of another form of the device of this invention, consisting of only one semi-circular open coil (120) facing upwards and supported by three tubular stands (bars) (121) that slide inside other three tubular stands of greater diameter (123); the arrangement of the tubular stands split in two sections, top and bottom, is necessary to regulate the height of the coil; a horizontal support (122) is located on the top end of the latter two stands; this support keeps the group of tubular stands resting on a thin disc-shaped base (124) in its position; the central stand conducts the power cable (125) of the coil. This device is light, occupies little space and is highly portable and suitable for traveling.

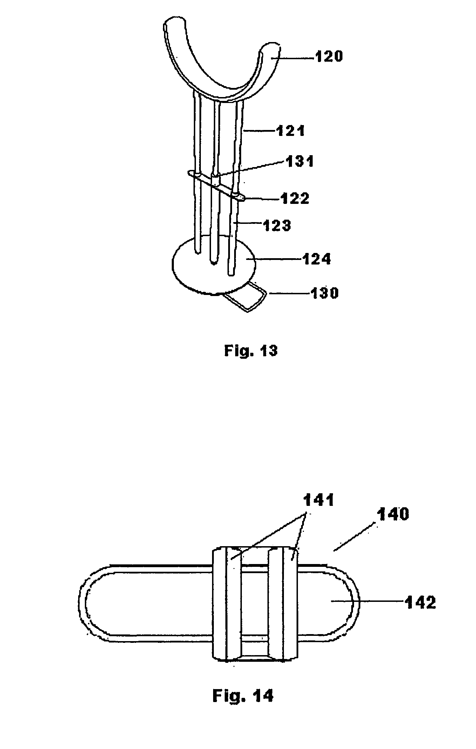

Figure 13 is a perspective view of the device of figure 12 that shows the coil (120) mounted on the group of three tubular stands (121) that slides upwards and downwards through the tubular openings (131) of the lower stands (123), which are held by the horizontal support (122) on the upper end and by the disc-shaped base on the lower end (124), and has a rectangular handle (130) that serves to move the device vertically into position.

Figure 14 is a top view (140) of one of the embodiments of this invention consisting of two circular doughnut-shaped Helmholtz coils (141) and one short plate (142) with rounded, semi-circular edges, where the patient can lie down comfortably.

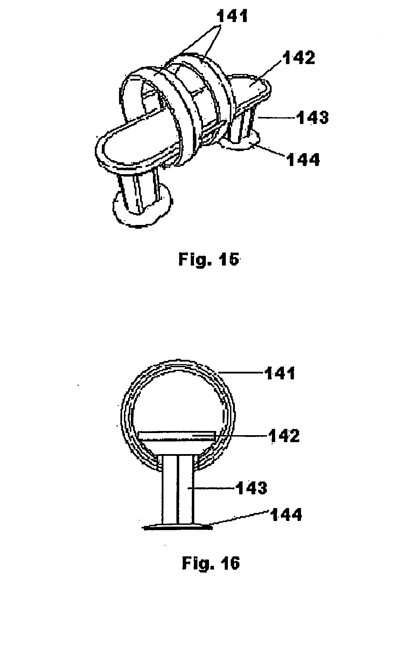

Figure 15 is a perspective view of the entire device of the form mentioned in figure 14, that shows the Helmholtz coils (141) in their final position, containing the plate inside (142); the plate is mounted on a structured base (not shown) that holds the coils and all the group of elements described above and is placed on two vertical stands at the ends (143), each one with an elliptical or oval platform or base (144), which is placed on the floor. The coils can be moved horizontally and parallel to the plate where the patient who is going to be irradiated with the magnetic field generated by the coils lies down.

Figure 16 is a cross section view of the device of figure 15 that shows the position of the coils (141) in relation to the plate (142); the coils are mounted on the stand (143) that is placed on the base (144) connected to the ground. The plate (142) resides in the internal space delimited by the coils, which can move parallel to the length of the plate.

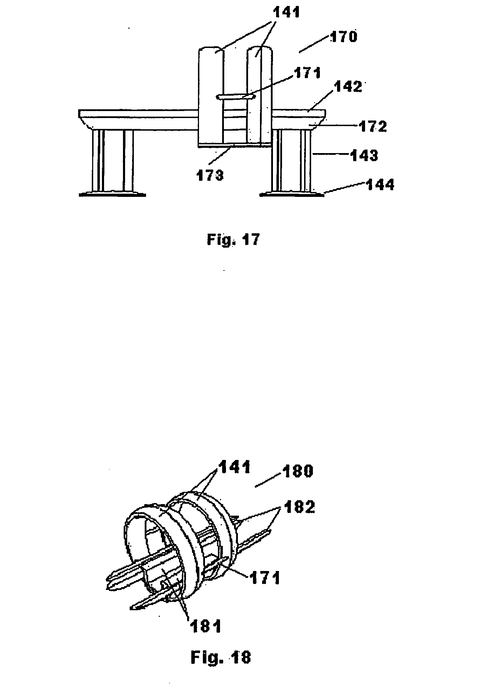

Figure 17 is a lateral view (170) of the device of figure 15 that shows the coils (141) moving horizontally and parallel to the plate (142) when the operator pushes the lateral handle (171), causing the coils to slide over the internal rail system (not shown) inside the base (172) of the longitudinal plate (142); the coils have a lower long support that is mounted on the structure (173) which is held from the outer edges of the coils; the whole group described above is held by the end stands (143) and the bases (144).

Figure 18 is a perspective view (180) of the coils (141) and its fastening (181) and sliding (182) system located on the lower part of the internal surface of the coils, which remain parallel and separated at an adequate distance by the lateral handles (171) and the angular fastening structures (181).

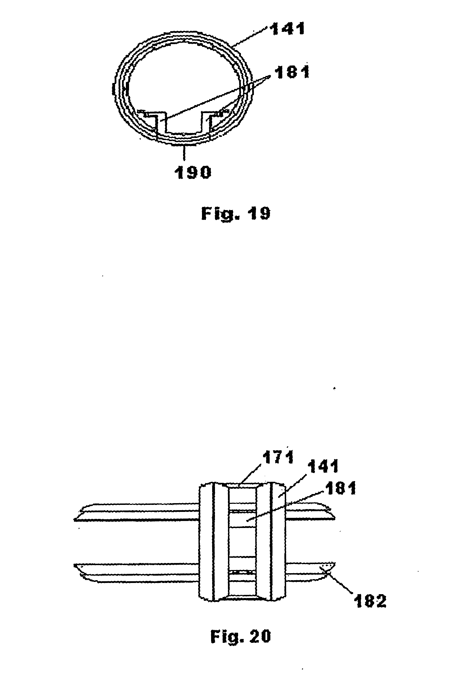

Figure 19 is a cross section view of the coils (141) and its fastening system (181) with an additional fixing element (190) located on the lower part of the internal surface of the coils.

Figure 20 is a top view of the group of coils (141), the fastening handles (171), the fastening structure (181) and the two pairs of sliding crossbeams (182), each made of two long parallel supports with a space between allowing a metallic flange to be fixed to other structural element where the group is mounted to slide.

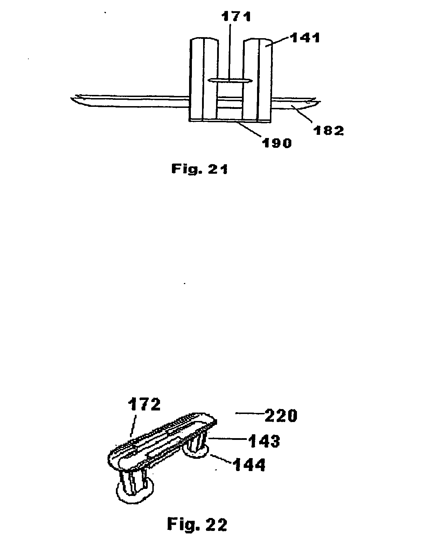

Figure 21 is a lateral view of the pair of coils (141) with their fastening handle (171), the additional fixing element (190) in the lower part of the outer edge of the coils; it also shows the profile of the crossbeams of the sliding system (182) of the coils when the handles (171) are pushed to place the coils in the adequate position to irradiate the magnetic field that they generate.

Figure 22 is a perspective view (220) of the base (172) of the plate equipped with the coupling elements to join it to the group of coils of figure 18, namely, the flanges (222) where the sliding elements (182) fixed to the coils connect so they can slide horizontally across the plate; the vertical longitudinal flanges located on the lower surface of the plate, which connect to the inferior end stands that support the coils and plate are also shown.

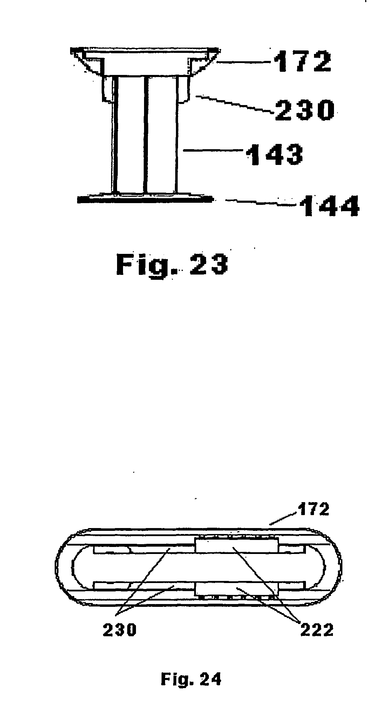

Figure 23 is a cross section view of the structural base (172) that shows the fastening elements (230) or longitudinal vertical flanges of the long base where the vertical stands connect (143) and that, together with its base (144) support the whole system.

Figure 24 is a top view of the structural base (172) of the plate that shows the detail of the coupling flanges (222) with the sliding crossbeams (182), the structural and fastening crossbeams together with the stands; said crossbeams are vertical flanges extending downwards. The external profile of the base that connects to the lower side of the plate is also shown.

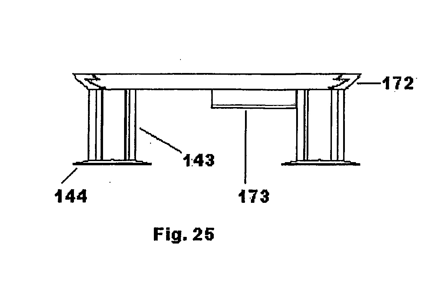

Figure 25 is a lateral view of the base (172) of the plate and the end stands (143) that rest on the stand bases (144); the auxiliary fastening structure of the coils (173) is also shown.

Two-Helmholtz coils configurations are needed for the proposed embodiments of the invention, namely, two 80 cm diameter coils with 40 cm of separation and two 30 cm diameter coils with 15 cm of separation; the cross section of the first configuration measures 5.25 X 5.25 cm with 625 turns of magnetic wire gauge 12, with an inductance of 628.77 MHz. The cross section of the second form measures 2.12 X 2.12 cm with 625 turns of magnetic wire gauge 20, organized in 25 layers of 25 turns each, with an inductance of 221 MHz.

This coil configuration produces very low frequency electromagnetic fields from 1 Hz up to 300 Hz, with intensities of 2 micro teslas and up to 0.8 militeslas.

The standard geometry of a Helmholtz coil consists of a pair of parallel coils separated a distance equal to their radius and in phase. The generated field is the sum of the fields generated by the two separate coils, resulting in a very uniform field between the coils; a field magnitude of 36.38 Gauss between the two 80 cm Helmholtz coils is required.

There are four basic configurations for a magnetic stimulator of this invention, namely:
A magnetic stimulator with a senoidal wave of 60 Hz, consisting of a 120-volts and 60 Hz power source (energy from domestic power lines), a switch, a transformer and the inductance coil.
A magnetic stimulator with a senoidal wave of 120 Hz, consisting of a 120 volts and 60 Hz power source, a switch, a transformer, a complete-wave rectifier and inductance coil.
A direct current magnetic stimulator (static field), consisting of a 120 volts and 60 Hz power source, a switch, a transformer, complete-wave rectifier, a capacitor and an induction coil.
A magnetic stimulator with a mechanical selector of field intensity, consisting of a 120 Volts and 60 Hz power source, a switch, a transformer with shunts, a mechanical switch and an induction coil.

To treat a patient suffering from skin injuries or internal wounds according to the method of this invention, the limb of the patient (for example a hand or part of an arm) can simply be introduced and partially extended across the tunnel formed by the coils. Then the power source is turned on to apply an electrical current to the solenoid or to the Helmholtz coils. The flow lines produced by the magnetic fields generated inside the tunnel include variable and static magnetic flow lines. It has been found that the treatment is more effective when the electromagnetic field is applied at extremely low frequencies ranging from more than 1.0 Hertz to less than approximately 300 Hz. The spectral content of the frequencies of the time-variable magnetic field is approximately 125 Hz higher, its harmonic resonance is also higher. These frequencies have a static field component with an intensity of approximately 0.3 to approximately 0.8 µT rms (root mean square) values, and these EMFs can be used individually or in combination with a homogeneous static field of approximately 40 to approximately 80 µT (approximately 400 to approximately 800 Gauss). Then, there are two static fields: one is generated along with the time-variable magnetic field and preferably has low magnetic flow density from a few microteslas up to a maximum of approximately 0.3 to approximately 0.8 mT (rms); the second field has a higher density and is generated by the permanent magnets with a magnetic flow density of approximately 40 to approximately 80 µT, equivalent to approximately 400 to approximately 800 Gauss. As indicated above, the application of time-variable magnetic fields with frequencies ranging from a few Hertz (more than one) up to less than approximately 300 Hz and static field components with an intensity of approximately 0.3 to approximately 0.8 mT, used individually or simultaneously with a homogeneous static field of approximately 40 to 80 µT or approximately 400 to approximately 800 Gauss, is an essential (critical) characteristic of the method of this invention.

The effects of wounds healing, regeneration of the nerve tissue, angiogenesis, regeneration of the muscle tissue, activation of the immune system cells and mother cells observed in the patients with this method and device are obtained through the use of the time-variable electromagnetic fields described in this application, either individually or in combination with static electromagnetic fields. Nevertheless, each separate component and in combination generally shows different degrees of action on the proliferation of peripheral mononuclear blood cells (PBMC). Said components show different degrees of action on the proliferation of cells, and this is demonstrated when different types of magnetic fields are applied to the PBMC of normal subjects (the 100% cellular proliferation control consists of PBMC stimulated to proliferate with phytohemagglutinin without the presence of any of the magnetic fields of this invention). The application of a combination of static fields and fields variable in time produced an increase of 11.1% of the PBMC proliferation (P<0.001 Test "T" of Student); the application of a magnetic field variable in time simply produced a reduction of 67% of the PBMC proliferation (P < 0.001 Test "T" of Student) and the application of a static field alone did not have a significant effect on the PBMC proliferation.

Preferably, the magnetic fields should de applied to the limb of the patient during a period ranging from approximately a few minutes up to approximately eight hours to obtain the maximum benefit from the treatment. The preferred exposure time was calculated so that a blood volume equivalent to the total blood volume of the patient is exposed to the magnetic field at least once. The frequency of exposure may vary from a maximum of approximately 30 minutes to approximately seven hours per day, up to a minimum of approximately 30 minutes per week, depending on the observed healing and scarring of the wound. Exposure times are reduced twenty times when applying the electromagnetic fields on the thorax because there is more blood circulating there than in the arm.

The following examples illustrate the effect of the EMFs according to the present invention, when applied on isolated peripheral mononuclear blood cells (PBMC) in order to regulate their proliferation patterns and their systemic effects in vivo; and the application of external time-variable magnetic fields, individually or in combination with an static magnetic field, to treat patients with chronic skin ulcers, chronic wounds, hypoperfused, but still viable myocardium, bone fractures and partial denervation.

The following examples illustrate the effectiveness of the method of this invention. However, these examples are only illustrative and are not meant to limit the scope of the invention.

### EXAMPLES

### IN VITRO EFFECT

The following experiments show the effect of electromagnetic fields (EMF), applied according to the present invention, on peripheral mononuclear blood cells (PBMC).

Example #1: *In vitro* effects of different electromagnetic fields on the proliferation of PBMC.

Peripheral mononuclear blood cells (PBMC) were drawn by puncturing a vein and then they were fractioned on a Ficoll gradient. The fraction containing the PBMC was washed 3 times and was again suspended in physiological saline, phosphate buffer up to a final concentration of 5 x 10⁶ cells/ml. Proportional parts of PBMC were drawn: aliquots of 0.2 ml of 5 x 10⁶ cells/ml, and 1 m/ml of phytohemagglutinin were taken and placed in Eppendorf tubes. Four subgroups were formed (three tubes per subgroup). Subgroup 1 was protected to avoid exposure to the electromagnetic fields. Subgroup 2 was exposed to the combination of time-variable electromagnetic fields and static fields according to this invention. Subgroup 3 was exposed only to the time-variable electromagnetic fields according to this invention and subgroup 4 was exposed only to the static field according to this invention. Four subgroups without phytohemagglutinin (control) were prepared and placed under the same experimental conditions.

All the tubes were incubated at a temperature of 37 °C in a CO, incubator with humidifier (CO, dual chamber water-jacketed incubator, Forma Scientific Mod. 3325). After an incubation period of 58 hours, 3H thymidine (final concentration of 5-6 /mM) was added to all the tubes, which were incubated for another 14 hours. All the tubes were harvested and washed by filtering their contents through glass fiber filter paper with a cell harvester (Skatron, Tranby, Norway) after 72 hours. The addition of 3H thymidine was determined by using a liquid scintillation counter (Beckmann Models 6000 SE). The average of the number of counts per minute for each sample was obtained in triplicate. The cellular viability was always greater than 90% determined by trypan-blue exclusion.

The combination of electromagnetic fields both variable in time and static produced an increment of 11% in the PBMC proliferation (P < 0.001 Student t-test). The time-variable electromagnetic fields alone produced a reduction of approximately 67% in the cellular proliferation (P < 0.001 Student t-test). The static fields did not have any effect on the PBMC proliferation. Therefore, the in vitro experiments demonstrated different effects on isolated PBMC for each magnetic field or their combination .

### IN VIVO EFFECT AND SAFETY STUDY

Example #2: Effect of the EMFs over bodily injuries.

### (Safety and tolerance study)

Because the electromagnetic fields go through biological tissues, they also produce *in vivo* effects over the circulating PBMC. The in vivo effects of the time-variable electromagnetic fields of this invention were observed, either individually or in combination with static fields, on chronic cutaneous wounds even after approximately thirty minutes to one hour of exposure to the EMFs generated by this invention on a bodily part located far from the zone of the wound. Over this period it is common to observe the wound drying and a reduction of its diameter.

The magnetic fields generated by the device described in this application are below the exposure limits considered safe by the World Health Organization (WHO). Nevertheless, due to the fact that it is a pioneering procedure, the following study was undertaken in order to illustrate the safety of the external application of the magnetic fields generated by this invention on human beings. After signing a voluntary consent letter to participate in the magnetic field protocol, each patient underwent through a complete clinical history, physical examination, blood chemistry, blood cell count and urine tests before being exposed to the magnetic field for the first time. An electrocardiogram was taken and the blood pressure was measured before, while and after being exposed to the electromagnetic field. Three volunteer groups were formed. The first group was exposed the electromagnetic fields (EMF) during 30 minutes per day, the second group during 30 minutes twice a week, and the third group during 30 minutes once a week. The three groups were followed up for one year without detecting adverse reactions.

Recently, more than a hundred patients have been exposed to external magnetic fields from one to seven hours per day, seven times a week, according to the method of this invention.

During the course of these treatments, no adverse or negative secondary effects were observed in any of these patients during or after the exposure period to the EMFs. The application of EMFs through the method and device of this invention did not show any effect on the electrocardiograms, even in patients with cardiac illnesses. This treatment did not produce any adverse effect on the motor or sensorial functions either.

### CONCLUSIONS

The aforementioned examples show that the application of external and non-invasive electromagnetic fields (EMF) according to the method of this invention, either individually or in combination with other medical or surgical treatment, heals the bodily wounds of patients that did not formerly respond to conventional medical or surgical treatment alone, or improve the effect of said treatments.

This way, according to the detailed description of the invention and the illustrative examples provided above, it is clear that the treatment is a clearly effective clinical method to improve the health and well being of the patients without producing adverse reactions of negative side effects.

The aforementioned information has been exemplified in patients with several illnesses and diseases, for example a history of partial denervation, internal or external wounds including injuries, areas with inadequate blood perfusion, hypoperfused, but still viable myocardium and the like; burns and ulcers either alone or accompanied by other disorders associated with the immune system or by the direct stimulation of the cells of the affected tissues.

The particular embodiments of the invention have been illustrated and described, for technical experts it will be evident that several modifications or changes can be made without departing from the intended scope. The attached claims intend to cover the aforementioned information so that all the changes and modifications are within the scope of the present invention.

## Claims

1. The use of external, non-invasive electromagnetic fields (EMF) with variable in time and static components of very low frequency, including frequencies ranging from approximately 2 Hertz up to less than approximately 300 Hz, and components of an static field from approximately 2 µT up to approximately 0.8 mT; the EMFs are applied during an effective period of time sufficient to promote the response to the pharmaceutical or surgical treatment, and to induce the healing of an illness or ailment associated with the injury; the application is local, either to the whole body or a bodily part suffering from the injury, the EMFs are applied on an area distant from the part or site of the wound in order to expose the complete blood volume of the patient to the EMFs at least once; said fields effectively rehabilitate and promote the regeneration of the skin and nerve tissue, increase angiogenesis, vasculogenesis, regeneration of nerve tissue, osteogenesis, reestablish the healing process of the wound, and have an analgesic anti-edema and/or anti-inflammatory effect; and the illness is selected from a group consisting of varicose veins, illnesses and conditions associated with pain, edema and inflammation, limited mobility, deep venous thrombosis, high blood pressure, injuries caused by trauma or surgery, lymphatic or venous failure, ulcers, vasculitis, partial denervation, bone fractures, bone unions, chronic injuries, chronic ulcers, varicose veins, rheumatoid arthritis, vascular necrosis, chronic wounds and burns.

2. The use of external, non-invasive electromagnetic fields (EMF) with variable in time and static elements of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin and nerve tissue on the ulcers located on the leg or foot of the patient, and selected from a group consisting of venous ulcers, arterial ulcers, decubital ulcers, deep ulcers caused by pressure, medium ulcers of the maleolus, artifact ulcers, ulcers caused by trauma or surgery and leg ulcers.

3. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin and nerve tissue on the wounds selected from the group consisting of surgical wounds, non-surgical wounds and surgical drainage.

4. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin and nerve tissue on the non-surgical wounds selected from the group consisting of compact wounds and decubital ulcers classified as "grade 5" in the Yarkony-Kirk scale.

5. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate the illness or condition associated with heart attack, stroke, partial denervation and/or neuronal apoptosis.

6. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin suffering from vascular necrosis associated with silicone implants.

7. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat a patient who has become resistant to the agent to treat the illness or condition, before the application of the magnetic field.

8. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin and nerve tissue on the pharmaceutical and surgical treatment selected from the group consisting of saphenectomy, skin grafts, surgery, closing of the wound, stitches, bandages, antihypertensive therapy, delivery of pharmaceutical compositions, nurse care and total cleaning of the bodily injury.

9. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to intensify the effect over the delivery of a composition including an agent selected from the group consisting of the delivery of pain blockers, anti-inflammatory agents, antibiotics, flavonoids, tissue plasminogen activator, acetylsalicylic acid, ketanserin, zinc oxide, hydrocolloids, alginates, foams, hydrofibers, gels, collagen mixtures, silver, compression systems, prostacyclin analogues, prostanoids, venotonic drugs, fibrinolytic and thrombolytic agents, beta blockers, Angiotensin-converting enzyme inhibitors and edible diets.

10. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat illnesses or bodily injuries accompanied by inadequate blood perfusion.

11. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat subjects selected from a group consisting of animals, human and non-human.

12. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat the illness or condition associated with hypoperfused myocardium.

13. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat the illness or condition associated with diabetes, diabetic foot, ankle fracture, ankle fracture accompanied by pain, edema and/or bone fracture.

14. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat the illness or condition associated with denervation, neuronal apoptosis or infection.

15. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat the illness or condition associated with a reduction of the blood flow in the brain, attention span or mental sharpness.

16. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to treat the illness or condition associated with devascularization.

17. The use of external, non-invasive electromagnetic fields (EMF) with variable in time and static components of very low frequency including frequencies ranging from approximately 2 Hertz up to approximately 300 Hz and static field components ranging from approximately 2 µT up to approximately 0.8 mT, according to claim 1, **characterized** because it is used to rehabilitate or promote the regeneration of the skin and nerve tissue, to intensify the collagen maturing process and increase the tensile strength during the healing process, to induce the proliferation and production of fibroblasts, angiogenesis and the healing of different tissues.

18. A device that generates an electromagnetic field variable in time with frequencies ranging from approximately 2 Hz to less than approximately 300 Hz and static field components ranging from approximately 2 µT up to approximately 0.8 mT, consisting of:
two Helmholtz coils shaped like a sound-sided quadrilateral having a diameter of 80 cm and separated 40 cm from each other, each one with 625 turns (25 layers of 25 turns each);
a rectangular base with wheelbarrows and a central inferior horseshoe-shaped opening, which is fixed to the top side of the coils; there are two wheelbarrows on the lower edges of each lateral side of said base;
a long rail-type base wit a central top slot and a distal stop at the end, over which the rectangular base with a central slot slides using wheelbarrows that roll on the lateral flanges of the rail;
a long top plate, located inside the internal space defined by the coils;
an outer shell shaped like a rectangular prism, with a longitudinal slot on the top edge of the long lateral sides;
two lateral handles located on the vertical sides of the coils.

19. A device that generates an electromagnetic field according to claim 18, also **characterized by** the fact that the coils slide horizontally if some force is applied to the lateral handles.

20. A device that generates an electromagnetic field according to claim 18, also **characterized by** the fact that the top plate connects to the outer shell edge that covers the longitudinal base.

21. A device that generates an electromagnetic field according to claim 18, also **characterized by** the fact that the top plate is longer than the outer shell of the longitudinal base and extends beyond the limit of the shell.

22. A device that generates an electromagnetic field according to claim 18, also **characterized by** the fact that the longitudinal lateral slots are fitted out so that the lower side of the pair of coils can pass through them and the coils can freely slide horizontally.

23. A device that generates an electromagnetic field according to claim 18, also **characterized by** the fact that all the elements except for the coils are made of a non-magnetic material.

24. A portable device that generates an electromagnetic field variable in time with frequencies ranging from approximately 2 Hz to less than approximately 300 Hz and static field components ranging from approximately 2 µT up to approximately 0.8 mT, consisting of:
two doughnut-shaped Helmholtz coils having a diameter of 30 cm with 15 cm of separation;
a semi-circular fixing element located in the space between the coils and parallel to them;
a top, long and rectangular fixing element located perpendicular to the coils in the internal surface between both coils; it has a displacement handle and a control panel on one of the ends.
a flat concave plate (sic) located on the lower section of the internal space defined by the coils, with two straight edges and two ends shaped like the section of an ellipse;
a flat and wide telescopic stand divided in a top section an a bottom section, fixed on a base, the top section slides into the bottom section;
a circular base with a ring-shaped edge on which four wheelbarrows with a regular angular distribution are mounted.

25. A device that generates an electromagnetic field according to claim 24, also **characterized by** the fact that all the elements except for the coils are made of a non-magnetic material.

26. A device that generates an electromagnetic field according to claim 24, also **characterized by** the fact that the displacement handle and the control panel of the device are located at the end of the top support and in front of the operator.

27. A portable device that generates an electromagnetic field variable in time with frequencies ranging from approximately 2 Hz to less than approximately 300 Hz and static field components ranging from approximately 2 µT up to approximately 0.8 mT, consisting of:
a semi-circular coil located on three tubular, telescopic stands (bars) divided in a top section and a bottom section; the top section slides inside the bottom section fixed on a disc-shaped base;
a fixing support for the stands, located at the top end of the lower stands;
a bottom positioning handle fixed on the bottom surface of the base.

28. A device that generates an electromagnetic field according to claim 27, also **characterized by** the fact that all the elements except for the coils are made of a non-magnetic material.

29. A device that generates an electromagnetic field variable in time with frequencies ranging from approximately 2 Hz to less than approximately 300 Hz and static field components ranging from approximately 2 µT up to approximately 0.8 mT, consisting of:
two doughnut-shaped Helmholtz , with a diameter of 80 cm and separated 40 cm from each other, each one with 625 turns (25 layers of 25 turns each);
a sliding and fixing structure, located on the bottom part of the internal surface of the coils;
a long top plate, with semi-circular ends, placed inside the internal space defined by the coils;
a long base with semi-circular ends for the plate; the base has an elongated ring shape, it has the fixing flanges to connect the fixing structure located inside the coils on the top side, , on the bottom side it has two longitudinal, vertical flanges that connect to the loading stands;
two vertical stands located on the ends of the base of the plate, on which the group formed by the coils and the plate is installed; two elliptical-shaped stand bases.

30. A device that generates an electromagnetic field according to claim 29, also **characterized by** the fact that all the elements except for the coils are made of a non-magnetic material.

31. A device that generates an electromagnetic field according to claim 29, also **characterized by** the fact that the coils have an internal fixing and sliding structure consisting of two angular fixing pieces and sliding crossbeams that connect to the flanges of the base of the plate.

32. A device that generates an electromagnetic field according to claim 29, also **characterized by** the fact that the top plate connects to its base, and the base connects to the pair of stands at the end, which support said base; the plate and the coils together with their fixing and sliding structure.

33. A device that generates an electromagnetic field according to claim 29, also **characterized by** the fact that the coils can slide inside the space defined between the vertical stands.

34. A device that generates an electromagnetic field according to claim 29, also **characterized by** the fact that the plate and its corresponding base do not stick out beyond the limits defined by the vertical stands.
